Europäisches Patentamt

**European Patent Office**

Office européen des brevets

(19)

(11) Numéro de publication: **0 261 032**
**A1**

(12)

# DEMANDE DE BREVET EUROPEEN

(21) Numéro de dépôt: **87402063.9**

(22) Date de dépôt: **15.09.87**

(51) Int. Cl.⁴: **A 61 L 2/20**
**C 12 N 7/06**

(30) Priorité: **16.09.86 FR 8612943**

(43) Date de publication de la demande:
**23.03.88 Bulletin 88/12**

(84) Etats contractants désignés:
**AT BE CH DE ES GB GR IT LI LU NL SE**

(71) Demandeur: **ASSOCIATION REGIONALE DE TRANSFUSION SANGUINE**
**10, rue Spielmann**
**F-67085 Strasbourg Cédex (FR)**

(72) Inventeur: **Schmitthaeusler, Roland**
**36, quai de l'Ill**
**F-67400 Ellkirch-Graffenstaden (FR)**

(74) Mandataire: **Peaucelle, Chantal et al**
**S.C. Ernest Gutmann - Yves Plasseraud 67, boulevard Haussmann**
**F-75008 Paris (FR)**

(54) **Procédé d'inactivation par ozonolyse de micro-organismes contaminants présents dans des matériaux biologiques essentiellement protéiques, les produits obtenus et leurs applications biologiques et analytiques.**

(57) Le procédé de l'invention comprend le fractionnement d'un matériau biologique donné et l'action d'un mélange $O_3/O_2$ renfermant de $10^{-3}$ à 150 µg/ml d'$O_3$.

EP 0 261 032 A1

## Description

PROCEDE D'INACTIVATION PAR OZONOLYSE DE MICRO-ORGANISMES CONTAMINANTS PRESENTS DANS DES MATERIAUX BIOLOGIQUES ESSENTIELLEMENT PROTEIQUES, LES PRODUITS OBTENUS ET LEURS APPLICATIONS BIOLOGIQUES ET ANALYTIQUES.

L'invention concerne un procédé d'inactivation par ozonolyse de micro-organismes contaminants présents dans des matériaux biologiques essentiellement protéiques, les produits obtenus, et leurs applications biologiques et analytiques.

On sait que les matériaux biologiques protéiques tels que le sang, les extraits ou broyats d'organes ou de tissus, d'origine animale ou humaine, renferment des micro-organismes qu'il convient d'inactiver pour les applications biologiques ou analytiques de ces matériaux.

Ils contiennent notamment des virus capables de franchir les filtres microporeux généralement utilisés pour leur stérilisation du point de vue bactériologique.

Jusqu'à présent, les méthodes connues d'inactivation in vitro des micro-organismes présents dans les matériaux biologiques s'appliquent de préférence à des préparations purifiées et font appel à des techniques physiques ou chimiques.

Parmi les méthodes les plus utilisées, on citera :
- l'action de la chaleur, généralement à 60°C, sur le matériau à l'état liquide ou sec, ce qui nécessite l'utilisation d'un agent protecteur contre les effets thermiques susceptibles de déstabiliser la ou les protéines du matériau ;
- l'action de détergents en présence de solvant, qui pose le problème de l'élimination du détergent qui peut s'avérer délicate lorsqu'on vise l'application thérapeutique des préparations traitées ou leur application pour la production de milieux de cultures cellulaires ;
- l'utilisation de rayonnement à haute énergie, qui peut entraîner la formation de radicaux libres et ainsi des polymérisations gênantes ;
- l'action d'un rayonnement ultra violet en présence de β-propiolactone composé qui ne peut être utilisé que dans des conditions spécifiques, ou encore
- la chromatographie d'interaction hydrophobe, utilisable essentiellement dans le cas de virus à enveloppe lipidique et dont la réalisation soulève fréquemment des difficultés techniques.

D'une manière générale, ces méthodes entraînent un effet dommageable sur les protéines de ces matériaux biologiques (perte d'activité de certaines enzymes, polymérisation, apparition de néo-antigènes, insolubilité partielle).

De plus, elles imposent l'élimination dans une étape supplémentaire, des produits à effet antiviral mis en oeuvre. Il faut également, avant par exemple tout emploi à des fins thérapeutiques, doser les résidus éventuels des agents d'inactivation virale, ce qui augmente le prix de revient du procédé.

La recherche par les inventeurs de méthodes d'inactivation de micro-organismes de mise en oeuvre plus aisée les a amenés à étudier l'action exercée par des agents oxydants tels que l'hypochlorite de sodium, l'acide peracétique et plus particulièrement l'ozone.

L'action de l'ozone entraîne une ozonolyse des composés organiques à double liaison, Carbone-Carbone, ce qui conduit à des sous-produits tels que des peroxydes qui agissent sur les enzymes d'oxydo-réduction présents dans les systèmes biologiques (glutathion, oxydases, peroxydases). Ces sous-produits agissent également au niveau des structures lipidiques, constituants principaux des membranes cellulaires. Elle entraîne également le blocage des récepteurs N-acétyl glucosaminique, souvent présents à la surface des virus, ce qui empêche la fixation des particules virales à la surface de la cellule cible du virus.

Il existe également un mode d'action indirect de l'ozone conduisant à une fragilisation des enveloppes des virus par fixation de l'ozone sur la phosphatidylcholine présente dans ces enveloppes. Il s'ensuit une diminution de la résistance de l'enveloppe du virus qui devient perméable aux métabolites et aux enzymes présents dans le milieu protéique infecté. Le virus est alors inactivé.

Les effets de l'ozone indiqués ci-dessus ont été observés sur des virus en solution aqueuse ou tamponnée (S. Rilling, R. Viebahn : Praxis des Ozon-Sauerstoff Therapie ; Ewald Fischer Verlang 1985).

On a également rapporté dans cet article l'utilisation de l'ozone pour hyperoxygéner un volume de sang anticoagulé, prélevé puis réinjecté au patient, afin de stimuler certains systèmes oxydo-réducteurs impliqués dans l'élimination de l'agent pathogène (ib. sup.)

Mais dans cette application, l'ozone agit de façon non spécifique vis-à-vis des éléments du sang, ce dernier jouant essentiellement le rôle de véhicule du gaz. L'amélioration de l'état du malade, d'ailleurs difficile à évaluer par les méthodes disponibles, nécessite une réponse métabolique de ce même malade. Il s'agit donc d'une action in vivo, inapplicable à l'inactivation in vitro de matériaux biologiques comme visée par l'invention.

Pour réaliser une inactivation in vitro par ozonolyse des micro-organismes contaminant un matériau biologique, il s'est avéré de manière inattendue que ce matériau devait être traité préalablement afin d'éliminer et/ou d'inactiver les systèmes oxydo-réducteurs susceptibles d'inhiber l'action oxydante de l'ozone.

L'invention a donc pour but de fournir un procédé d'inactivation des micro-organismes contaminant des matériaux biologiques de nature essentiellement protéique, de grande efficacité, tout en préservant les propriétés fonctionnelles des protéines.

Elle vise également à fournir un procédé de mise en oeuvre simple impliquant l'utilisation d'un agent oxydant connu, à savoir l'ozone.

L'invention vise en outre à fournir des produits utilisables en biologie ou à des fins analytiques, ou encore pour la production d'un milieu de culture cellulaire dépourvu de contaminants plus spécialement de virus ou de champignons.

Le procédé selon l'invention est caractérisé en ce qu'il comprend :

a/ - le fractionnement du matériau biologique de départ par précipitation ou extraction afin d'éliminer et/ou de déséquilibrer les systèmes d'oxydo-réduction susceptibles d'inhiber l'action oxydante de l'ozone vis-à-vis des micro-organismes contaminants et,

b/ - l'action d'un mélange $O_3/O_2$ préparé à partir d'oxygène, de composition contrôlée, renfermant $10^{-3}$ µg/ml à 150 µg/ml d'$O_3$, de préférence 10 à 50 µg/ml.

De manière avantageuse, on constate que l'élimination des systèmes d'oxydo-réduction précités, permet de mettre à profit l'action spécifique de l'ozone en tant qu'inactivateur de micro-organismes. Certains de ces systèmes oxydo-réducteurs dont l'effet est une neutralisation de l'action de l'ozone sont par exemple des oxydoréductases ou des peroxydases.

Suivant une disposition supplémentaire de l'invention, le matériau biologique de départ est utilisé sous forme liquide ou pulvérulente.

Ainsi selon une première étape supplémentaire, on fait barboter dans le milieu biologique liquide ou on insuffle dans le milieu biologique pulvérulent un courant $O_3/O_2$ jusqu'à saturation en $O_3$ dudit milieu.

Il est avantageux de refroidir le milieu biologique liquide ou solide, à une température compatible avec la phase liquide quand le milieu est liquide pour augmenter la solubilité d'$O_3$.

Ce refroidissement est effectué plus spécialement à une température de l'ordre de 0°C à 15°C dans le cas d'un milieu liquide et de l'ordre de -30°C à 15°C dans le cas d'un milieu solide.

Pour compléter l'inactivation et la stabilisation du produit final, on a recours à une étape supplémentaire de lyophilisation ou d'atomisation du milieu traité.

Conformément à un mode particulier de réalisation de l'invention, on a recours à une étape de traitement par des fréquences ultrasonores de l'ordre de 20 à 37 KHz en opérant à une puissance d'environ 40 à 1000 W appliquée simultanément au barbotage ou à l'insufflation de l'ozone. Ce traitement par les ultrasons, facilite la disruption des enveloppes de virus tels que le Poliovirus I.

Le matériau biologique de départ mis en oeuvre dans le procédé de l'invention est un matériau de nature protéique constitué par des extraits tissulaires, notamment de placenta, des broyats d'organes purifiés, des dérivés sanguins tels que le sérum, le plasma ou le sang complet. Ces matériaux sont d'origine humaine ou animale.

Selon un mode préféré de réalisation de l'invention, on utilise comme matériau de départ la fraction cryoprécipitée et/ou la fraction surnageante telles qu'obtenues par congélation d'un plasma à une température d'environ -20° C. puis décongélation lente jusqu'à 2°C à 4°C. Cette technique de congélation et décongélation largement utilisée pour obtenir le produit appelé cryoprécipité est décrite dans:Striker MH, Waldmann AA. Blood Fractionation, dans : Kirk RE, Othmer DF, ed. Encyclopedia of Chemical Technology New York : John Wiley and Sons 1978 : 25-61.

On rappelle que le cryoprécipité présente la composition suivante :
- fibrinogène 15 g/l
- facteur XIII, 200 $\pm$ 50 µ/ml
- la fibronectine 7,5 $\pm$ 0,5 g/l
- protéines totales 25 $\pm$ 5 g/l
- protéines coagulables 50 à 60%

Le temps de coagulation avec 5 u/ml de thrombine est de 60 $\pm$ 10 sec.

Le traitement de congélation puis décongélation du plasma permet d'agir au niveau des systèmes oxydo-réducteurs susceptibles d'inhiber l'action oxydante de l'oxone. Grâce à l'élimination ou au déséquilibre de ces systèmes, il est possible d'obtenir une action spécifique de l'ozone sur les micro-organismes contaminants.

D'une manière générale, les micro-organismes sont constitués par des virus ou des champignons. L'action virucide de l'ozone opère ainsi en particulier sur le virus HIV (agent causal du sida) le phage T4, le virus VSV (virus de la stomatite vésiculeuse).

L'invention vise également les matériaux biologiques tels qu'obtenus après traitement par l'ozone. Les produits sont alors dépourvus des systèmes oxydo-réducteurs inhibiteurs vis-à-vis de l'action de l'ozone. Is sont caractérisés en ce qu'ils sont dépourvus de micro-organismes contaminants, ce qui permet de les utiliser directement à des fins biologiques ou analytiques ou pour la production de milieux de culture cellulaire.

D'autres caractéristiques et avantages de l'invention apparaîtront dans les exemples qui suivent relatifs à l'inactivation de virus ajoutés dans des préparations de cryoprécipité et de surnageant de plasma.

Dans les exemples qui suivent, des séries témoins infectées non traitées par l'ozone (témoins positifs) sont introduites, ainsi que des témoins négatifs, non inoculés, afin de faciliter l'interprétation des résultats.

## EXEMPLE 1

Une suspension de protéines à la concentration de 20 g/l contenant un mélange de fibrinogène, fibronectine, facteur XIII, albumine et immunoglobulines G, obtenue par resuspension d'un cryoprécipité, est additionnée d'une suspension virale contenant du phage lambda pour arriver à une concentration finale dans le mélange de $7.10^7$ particules virales par ml.

On congèle puis on lyophilise la suspension ainsi contaminée. Un mélange $O_3/O_2$ contenant 12 µg/ml d'$O_3$ et obtenu à partir d'un générateur branché sur une bouteille d'$O_2$, est insufflé dans un flacon contenant le cryoprécipité désséché et pulvérisé, refroidi à 0°C pendant 15 minutes.

On remet en suspension au volume original du cryoprécipité, dans un milieu tamponné puis on titre les virus résiduels par la méthode des plages de lyse sur étalement de bactéries E. Coli Q 358. Le taux de virus survivants montre une diminution de 2 $\log_{10}$

par rapport au flacon témoin lyophilisé mais non traité par O$_3$.

EXEMPLE 2

Un plasma frais obtenu sur anticoagulant est infecté par une suspension de retrovirus HIV correspondant à une activité de réverse transcriptase (RT) de 1,4 10$^6$ cmp/poche (la RT est l'enzyme essentielle pour la répplication des retrovirus).

Une partie du plasma contaminé est traitée directement par l'ozone, puis congelée et décongelée lentement. Malgré ces conditions habituelles pour la formation d'un cryoprécipité, celui-ci n'est pas obtenu. La détermination des virus résiduels de ce plasma est faite en rapport avec l'activité de RT, qui est proche de 130.000 cpm/ml au 13ème jour après mise en incubation dans une culture de lymphocytes. La valeur de l'activité de RT du plasma témoin étant de 175 000 cpm/ml on constate que l'inactivation n'est pas significative.

L'autre partie du plasma infecté puis congelé, est décongelée pour obtenir un cryoprécipité et un surnageant séparé du cryoprécipité. Ce surnageant est traité par barbotage d'un mélange O$_3$/O$_2$ à 12 µg/ml d'O$_3$ pendant 30 minutes à 0°C. L'activaté RT titrée dans une culture de lymphocytes montre un taux nul par rapport au surnageant témoin non traité par l'ozone (176 000 cpm/ml au 13ème jour).

Le cryoprécipité, remis en suspension dans un tampon citrate-salin, est divisé en deux parties.

La première partie est traitée à l'état liquide par le mélange gazeux O$_3$/O$_2$ selon le procédé décrit, puis congelé et lyophilisé.

Le produit sec est reconstitué par 10 ml d'eau distillée et 0,6 ml sont prélevés et mélangés à 0,6 ml de thrombine à 5 u/ml. Le caillot formé est mis à incuber dans 20 ml de milieu contenant 5.10$^5$ lymphocytes.

On ne met en évidence aucune activité RT, même au 20ème jour après la mise en culture.

La deuxième partie du cryoprécipité est lyophilisée, puis, après pulvérisation, traitée par insufflation du mélange O$_3$/O$_2$ à 12 µg/ml d'O$_3$, pendant 30 minutes à 0°C.

On dissout à nouveau le produit sec traité mais on ne met en évidence aucune activité RT, même après 20 jours de mise en culture.

Afin d'étudier l'effet du traitement par l'ozone, on réalise l'expérience suivante :

- Le cryoprécipité remis en solution dans un tampon contenant de l'aprotinine à 2000 unités inhibitrices de protéases (UIP) possède des propriétés particulières d'adhésivité et de coagulabilité. On connaît son utilisation sous forme de colle biologique et le contrôle d'efficacité de cette colle, par la mesure de la force de traction nécessaire à l'arrachement d'une pièce de peau de souris, encollée sur le dos de l'animal sacrifié.

Sans traitement, la valeur moyenne d'un échantillon de cryoprécipité utilisé pour collage est de 116 g/cm$^2$. Après traitement à l'état liquide, la force d'arrachement est de 122,5 g/cm$^2$.

Ceci montre que le cryoprécipité n'est pas modifié par le traitement à l'ozone, quant à ses propriétés adhésives.

Revendications

1/ - Procédé d'inactivation par ozonolyse de micro-organismes contaminants présents dans des matériaux biologiques essentiellement protéiques, caractérisé en ce qu'il comprend :

a/ le fractionnement du matériau biologique de départ par précipitation ou extraction afin d'éliminer et/ou de déséquilibrer les systèmes d'oxydo-réduction susceptibles d'inhiber l'action oxydante de l'ozone vis-à-vis des micro-organismes contaminants et,

b/ l'action d'un mélange O$_3$/O$_2$ préparé à partir d'oxygène de composition contrôlée, renfermant 10$^{-3}$ à 150 µg/ml d'O$_3$.

2/ - Procédé selon la revendication 1, caractérisé en ce qu'on utilise le matériau biologique de départ sous forme liquide ou pulvérulente, et qu'on fait barboter ou qu'on insuffle le mélange O$_3$/O$_2$ de préférence sous agitation jusqu'à saturation du milieu liquide ou solide.

3/ - Procédé selon la revendication 2, caractérisé par l'étape supplémentaire de refroidissement du milieu liquide ou solide, à une température compatible avec, pour le milieu liquide, son maintien en phase liquide notamment de l'ordre de 0 à 15°C, pour le milieu solide la température étant de l'ordre de -30 à 15°C.

4/ - Procédé selon l'une quelconque des revendications 1 à 3, caractérisé par l'étape supplémentaire de lyophilisation ou d'atomisation du milieu traité.

5/ - Procédé selon l'une quelconque des revendications précédentes, caractérisé en ce qu'il comporte une étape réalisée simultanément au barbotage ou à l'insufflation de traitement du milieu par des fréquences ultrasonores de l'ordre de 20 à 37 KHz, en opérant à une puissance d'environ 40 à 1000 W.

6/ - Procédé selon l'une des revendications 1 à 5, caractérisé en ce qu'il est appliqué à un matériau biologique de départ constitué par des extraits tissulaires ou des broyats d'organes purifiés, d'origine humaine ou animale.

7/ - Procédé selon l'une des revendications 1 à 5, caractérisé en ce qu'il est appliqué à un matériau biologique de départ constitué par du sang ou un dérivé sanguin tel que le sérum.

8/ - Procédé selon l'une des revendications 1 à 5, caractérisé en ce qu'il est appliqué à la fraction cryoprécipitée (CP) et/ou à la fraction surnageante (PDC) issues d'un plasma congelé puis décongelé lentement.

9/ - Procédé selon l'une quelconque des revendications précédentes, caractérisé en ce que les microorganismes présents dans le milieu sont des virus ou des champignons.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 325 568  (E. SANDER)<br>* En entier * | 1 | A 61 L    2/20<br>C 12 N    7/06 |
| A | EP-A-0 086 071  (REGENTS OF THE UNIVERSITY OF CALIFORNIA)<br>* Page 4, lignes 25-38 * | 1 | |
| A | FR-A-2 339 621  (RECHERCHES HEMATOLOGIQUES)<br>* Page 9, lignes 1-15 * | 1 | |
| A | EP-A-0 131 740  (NEW YORK BLOOD CENTER)<br>* Revendications 1,13 * | 1 | |
| A | WO-A-8 304 371  (R.H. PURCELL et al.)<br>* Pages 6-9 * | 1 | |
| P,X | EP-A-0 199 479  (IMMUNOLOGICS)<br><br>*  Colonne  2,  lignes  28-54; revendications 1,2,6 * | 1-4,7-9 | |

DOMAINES TECHNIQUES RECHERCHES (Int. Cl.4)

A 61 L    2/20

Le présent rapport de recherche a été établi pour toutes les revendications

| Lieu de la recherche<br>LA HAYE | Date d'achèvement de la recherche<br>26-10-1987 | Examinateur<br>PELTRE CHR. |
|---|---|---|